# EUROPEAN PATENT APPLICATION

(11) **EP 1 967 174 A2**
(43) Date of publication of application: **10.09.2008**
(21) Application number: 08250695.7
(22) Date of filing: 29.02.2008
(51) Int. Cl.: A61K 8/31, A61K 8/35, A61Q 19/04

(54) **A self-tanning composition, use of DHA in combination, use of DHA in combination with mineral oil, and a skin self-tanning method**

(30) Priority: 01.03.2007 BR PI0700674
(71) Applicant: Johnson & Johnson Industrial Ltda., 12237-350 Sao José dos Campos SP (BR)
(72) Inventor: Masiero, Silvana, 12245-820 Sao Jose dos Campos SP (BR); Tazinassi, Mariane Giroto, 12230-090 Sao Jose dos Campos SP (BR); Pedreca, Maria Goretti Delfino, 12233-370 Sao Jose dos Campos SP (BR); Oliveira, Sergio Luiz, 12227-660 Sao Jose dos Campos SP (BR)
(74) Representative: Warner, James Alexander

(57) **Abstract**

The present invention relates to a cosmetic and/or dermatological self-tanning composition containing reduced dihydroxyacetone (DHA) content and that uses the synergistic tanning effect of the combined use of DHA and mineral oil in obtaining an even tan, without stains and without producing skin dryness. The composition exhibits an improved stability by virtue of the lower DHA concentration and, through combination of specific components present in its composition, provides skin hydration together with self-tanning.

## Description

### Field of the Invention

The present invention relates to a self-tanning composition that, through a specific combination of its components, provides a natural tan built gradually, which does not stain the skin, does not make it dry and still provides moisturizing thereof. The composition results from the unexpected synergistic effect of the dihydroxyacetone and mineral oil that provide a tanning degree similar or superior when compared to traditional products, without the drawbacks resulting from application thereof.

### Description of the Prior Art

The self-tanners are a safe, rapid and effective alternative for obtaining healthful skin coloration. These products have been more and more sought after, chiefly in countries where the presence of sunlight is scarce. By using self-tanners one can obtain the desired tan throughout the year, without skin damage preventing the risks of excessive exposure to sunlight and the use of tanning chambers. Recently, the scientific community alerts for the risks involving artificial tanning on tanning chambers. According to Dermatologists and researchers, irresponsible uses of tanning chambers may contribute to the increase of skin cancer and induce premature skin aging, since the equipment used in artificial tanning emit mainly Ultra Violet radiation Type A (UV-A), which is also responsible for skin cancer and skin photoaging. Premature skin photoaging is characterized by dark, clear or reddish spots, dry, thick and wrinkled skin, making a person look older than she/he really is.

In this context, the use of self-tanners is considered a safe and practical alternative method for obtaining a healthful color, which is maintained for long periods through regular use thereof.

Tanning effects of most self-tanning products are caused by dihydroxyacetone (DHA), which reacts with dead cells' amino acids of the outermost layer of the skin, producing a yellowish pigment called melanoidine. The process of generating the tanned color is quite different from that caused by ultraviolet rays emitted by the sun or by artificial tanning chambers, which stimulate the production of melanin, but accelerating early aging and can induce skin cancer.

DHA can be used in a number of self-tanning cosmetic compositions. DHA concentrations in these compositions varies according to the intensity of the desired tan and usually ranges from 2% to 8% by weight. Depending on the product formulation and skin type, tanning effects appears after 2-3 hours of use.

Despite of being considered non-toxic, the self-tanning compositions containing DHA, chiefly those containing high DHA-concentrations, can cause skin dryness and the formation regions with different tanning degrees due the difference of skin composition in different parts of the body or due to the non-uniform application of the product containing DHA. Self-tanning compositions containing DHA also present stability problems and may form, according to the tanning conditions, by-products that have tanning effect, but produce different tanning colors from the desired one.

Aiming to improve the stability of self-tanning compositions containing DHA, document Pl 0412149-0 presents cosmetic kits comprising at least two separate components that are mixed together at the moment of application. Said "at least two separate components" comprise a self-tanning agent including DHA in one component and a reactive active for skin treatment in the other. The separation of the components of the composition was the strategy used for maintaining the properties of the formulation described.

Document P10205469-8 relates a cosmetic and/or dermatological composition, more particularly designed for artificial tanning and/or darkening of the skin and that comprises, in a cosmetically acceptable carrier, at least one amino-substituted 2-hydroxybenzophenone derivative and at least one self-tanning agent including DHA. The presence of the amino-substituted 2-hydroxybenzophenone derivative improves the stability of the tanning agent, improving its self-tanning capability when applied to the skin.

Document W02005/077327 describes self-tanning compositions containing dihydroxyacetone and characterized by containing more then 5% by weight of glycerin. The compositions described include lotions, creams and self-tanning foam containing, in addition to dihydroxyacetone and glycerin, surfactants, moisturizers agents, fatty alcohols, EDTA, antioxidants, mineral oil and other additives that, according to said document, promote tanning, forming a of healthful color appearance closer to the natural tanned skin than the traditional compositions.

The present invention describes self-tanning compositions that are stable, producing a natural and uniform tan also promoting skin hydration. Such compositions use the unexpected synergistic tanning effect of the action of DHA and mineral oil, for the purpose of decreasing the DHA concentration in the composition and, as a result, the disadvantages coming from the use thereof at high concentrations.

The compositions described in the prior-art documents mentioned above, as well as in other publications, do not consider, at any moment, the use of the unexpected synergistic tanning effect of the present invention, for obtaining compositions with reduced DHA concentrations.

### Objectives of the invention

One objective of the present invention is to provide a self-tanning cosmetic composition containing DHA and that does not have the drawbacks of the presently available compositions.

Another objective of the present invention is to use DHA in combination with mineral oil in preparing a self-tanning cosmetic and/or dermatological composition that has performance similar or superior to that of the presently available compositions.

A further objective of the present invention is to provide a skin self-tanning method through application of a cosmetic and/or dermatological composition containing DHA and mineral oil.

### Brief description of the invention

The presente inventions relates a self-tanning cosmetic and/or dermatological composition containing reduced DHA concentration comprising the synergistic effect not expected from the joint use of DHA and mineral oil in obtaining a uniform tan, without stains, or skin dryness The composition has improved stability due to the lower DHA content and, through combination of specific components in its composition, provides skin hydration simultaneously to the self-tanning.

### Description of the figures

Figure 1 Color brightness variation by days of application of 4 different self-tanning formulations.
Figure 2 Red color variations by days of application of 4 different self-tanning formulations.
Figure 3 Yellow color variations by days of application of 4 different self-tanning formulations.
Figure 4 Chromatic variations (ΔC) considering the combined variation of the red and yellow colors by days of application of 4 different self-tanning formulations.
Figure 5 Total combined variation (ΔE) considering the measurements of color brightness, variation of red color and variation of yellow color by days of application of 4 different self-tanning formulations.
Figure 6 skin hydration corneometry variation by 2 self-tanning and control formulations.
Figure 7 Color brightness variations by the days of application of 2 different self-tanning formulations.
Figure 8 Red color variations by the days of application of 2 different self-tanning formulations.
Figure 9 Yellow color variation by the days of application of 2 different self-tanning formulations..
Figure 10 Chromatic variations (ΔC) considering the combined variation of the red and yellow colors by the days of application of 2 different self-tanning formulations.
Figure 11 Total combined variation (ΔE) considering the measurements of color brightness, variation of red and yellow colors and variation of the yellow color by the days of application of 2 different self-tanning formulations.

### Detailed description of the invention

Dihydroxyacetone is a simple three carbon sugar, a physiological product of the organism, formed and used during glucolysis. Dihydroxyacetone used in self-tanning formulations is obtained by fermentation using *Glu-conobacter oxydans.*

The dihydroxyacetone action site is the corneous stratum on skin. The self-tanning process takes place in the outermost layers of the epidermis, through Maillard reaction, responsible for the tanning that occurs between the amino group of the skin keratin and the hydroxyl group of DHA, forming melanoidine, a brown color product..

The tanning intensity depends on the concentration of DHA - The more DHA applied, darker the tanning will be. DHA purity influences directly the formulation, and product quality, Pure DHA decreases its concentration in the formulation, provides a better pH for Maillard reaction, and provides more rapid and efficient tanning

The pH exerts an important function in tanning. The optimum pH for the Maillard reaction ranges from 5 to 6, which is the normal pH skin. DHA pH stability ranges from 3 to 4. However, considering the tendency to pH decrease, some compositions have an initial pH of 5.0.

Dihydroxyacetone, when applied to the skin, is absorbed by the corneous stratum, the outermost corneous layer of the epidermis. The systemic absorption of DHA, is considered insignificant. Skin Coloration obtained by using dihydroxyacetone-based products is closer to natural tanning, when compared with that obtained with older products. The tonalities obtained can be acceptable for people with intermediate skin color ton with respect to those having a very clear or very dark skin.

The advantage of cutaneous pigmentation induced by dihydroxyacetone is that it cannot be removed by perspiration, dives or baths. The "tanning" provided by DHA will only be removed by skin scaling. Factors like use of DHA with a higher degree of purity and the improvement of the compositions, with adequate pH values, enable a more rapid tanning, by employing lower DHA concentrations.

After application of the dihydroxyacetone preparations, one can observe a alteration of the skin coloration within 2 - 3 hours. Most people can achieve the desired appearance of tanned skin with two to four successive applications, and the desired application can be maintained through continuous applications every two to four days.

By virtue of the action mechanism, which is characterized by the reaction of dihydroxyacetone with the proteins of the corneous stratum, cutaneous pigmentation is directly correlated with the thickness and compaction of the corneous stratum. Thus, more keratinized areas like knees elbows, palm plantar region become pigmented more intensely than other skin areas in which the corneous stratum is less thick. In comparison with the ends, the face requires smaller amounts of product to achieve the desires appearance, but the reapplications should be more frequent.

Following the use of dihydroxyacetone as a self-tanner, the use thereof was also introduced for the purpose of camouflaging the depigmented lesions of patients with vitiligo.

In comparison with the commercially available cover-ups, dihydroxyacetone has the disadvantage of being resistant to water, presenting high substantively to the skin and, besides, pigmentation provided by DHA does not stain clothes. DHA consists of a safe and effective cosmetic method for masking vitiligo.

In the literature there are some papers reporting the use of dihydroxyacetone in vitiligo, demonstrating satisfactory results, DHA being considered a practical and mode of treatment that is quite more accepted by the patients. DHA is a valid option for the cases of vitiligo, since it does not interfere with the synthesis of melanin, but promotes skin pigmentation through the Maillard reaction between the amino group of the skin protein (keratin) and the hydroxyl group of DHA, forming a product of brown coloration, melanoidine, which, although exhibiting the same visual effect as melanin, is chemically different from it.

Although the use of DHA may be considered non-toxic in self-tanning compositions, it may make the skin very dry and cause a stained tan due to a non-uniform spread over the body or due to the different keratin compositions in various regions of the body. In addition, compositions containing DHA may present instability problems and, depending on the nature of the medium in which they are contained, they may decompose as time passes. This instability is reflected in the formation of a yellow coloration after prolonged storage of compositions containing DHA.

Within this context, the obtainment of compositions with lower DHA content and that provide a satisfactory tanning has become advantageous and interesting. The composition described in this invention exploits the unexpected synergistic effect of the intense and uniform tanning resulting from cosmetic and/or dermatological compositions containing mineral oil and low DHA content. The use of such compositions provides tanning effects similar or superior to those resulting from traditional compositions containing higher DHA contents, without the drawbacks of instability of these compositions, as well as the drawbacks caused by application of products with high DHA content, such as making the skin very dry and the appearance of stains due to uneven application of the product or application thereof in keratinous regions of the body.

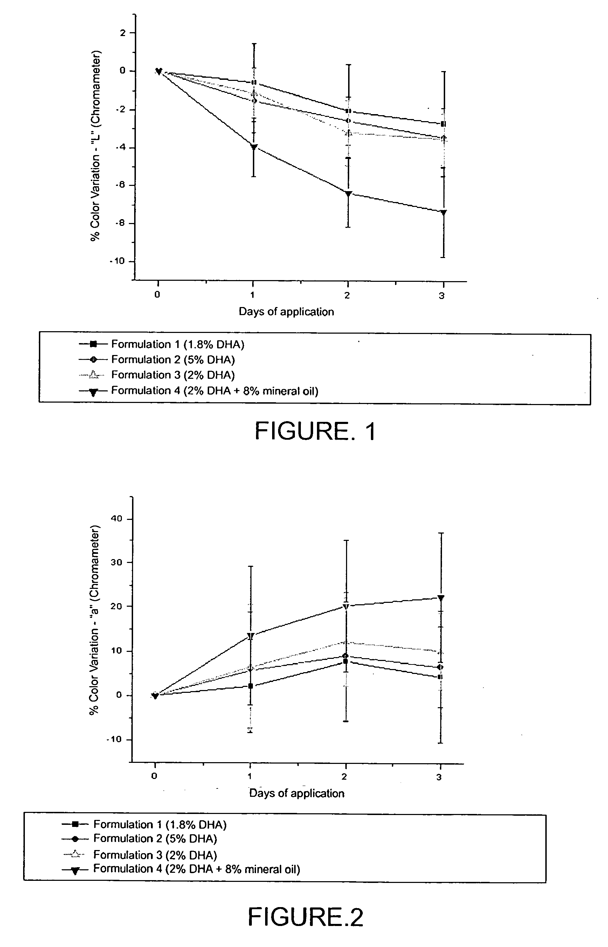

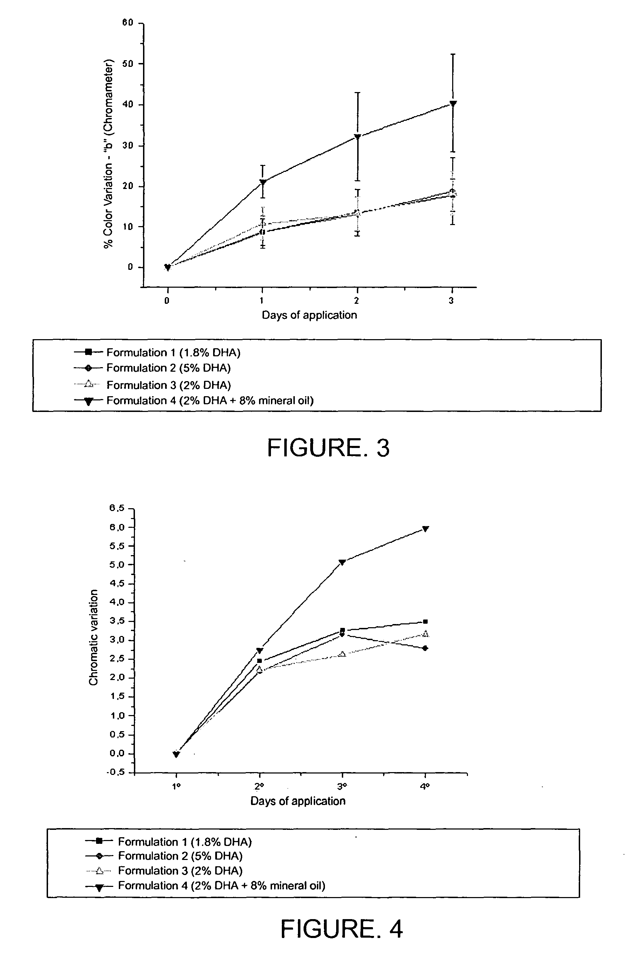

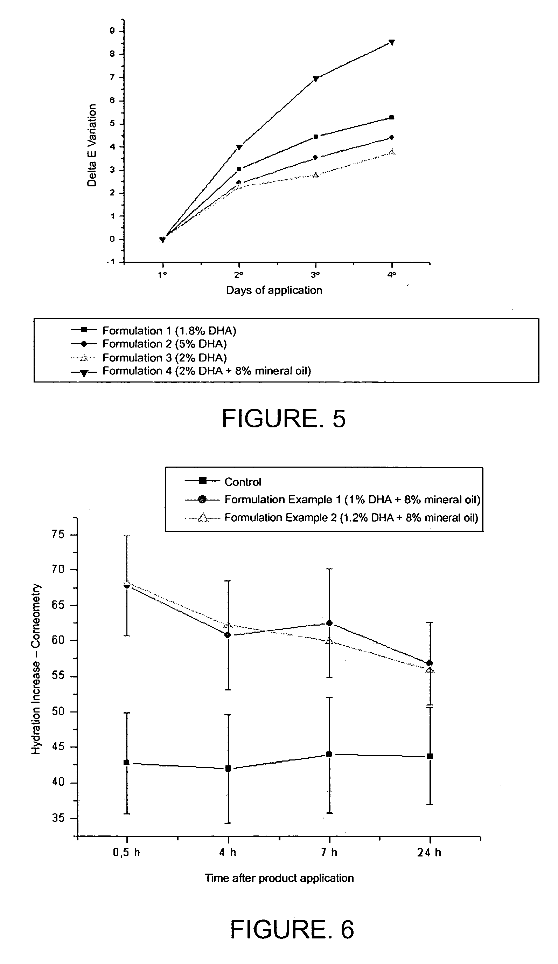

The unexpected synergistic tanning effect using the combination of DHA and mineral oil, according to the present invention, is shown through experiments carried out where the evolution of the color as a function of the days of application of the self-tanning was measured by calorimeter (Chromameter CR-200 - Minolta). For the experiments carried out, one used composition containing different concentrations of DHA in comparison with the composition containing 2% DHA and 8% mineral oil, according to the present invention. The amounts of DHA and mineral oil present in each formulation, as well as the variation of the parameters analyzed for each composition, can be seen in figures 1 to 5. One carried out, through calorimeter, measurements of variation of color brightness (figure 1), variation of red color (figure 2), variation of yellow color (figure 3), chromatic variation (ΔC), considering the combined variation of the red and yellow colors (figure 4) and total combined variation (ΔE), considering the measurements of color brightness, variation of red color and variation of yellow color (figure 5). As shown in said figures, the formulation 4 containing 2% DHA and 8% mineral oil exhibited, for all parameters analyzed, better tanning performance as compared with the other formulations. The formulation 4 exhibited greater variation of the color brightness (figure 1), greater enhancement of red color (figure 2), greater enhancement of yellow color (figure 3), greater chromatic variation (ΔC) (figure 4) and greater total combined variation (ΔE) (figure 5) when compared with the other compositions free from mineral oil. This unexpected synergistic effect of DHA in the presence of mineral oil enables that formulations containing DHA contents lower than those traditionally used can be employed in self-tanning compositions without decreasing their tanning effect.

The composition described in the present invention comprises, at most, 4% DHA and, at least, 6% mineral oil. Preferably, the composition described comprises, at most, 2% DHA and, at least, 8% mineral oil. More preferably, the composition of the present invention comprises 1.2% by weight DHA and 8% by weight mineral oil. The preferred composition for tanning a skin from normal to dark is that which comprises 1.2% by weight DHA and 8% by weight mineral oil. When used for tanning normal-to-clear skin, the composition of the invention preferably comprises 1.0% by weight DHA and 8% by weight mineral oil.

The composition of the present invention further comprises a wetting agent at concentration ranging from 0.1 to 10% by weight. The wetting agent preferably employed is glycerin. The preferred concentration of glycerin employed is of 2.5% by weight.

In addition to the components mentioned, the composition of the present invention may further comprise other components, such as carriers, preservatives, substances added for adjusting and/or maintaining the pH, viscosity agents, emulsifiers, emollients, perfumes and other materials compatible with the tanning composition.

Hereinafter 2 examples of compositions that can be made with the teachings of the present invention are given. In the compositions described, the DHA content was reduced to 1% by weight and 1.2% by weight with the use of 8% by weight of mineral oil. The examples of compositions described herein should be interpreted as preferred embodiments of the invention and should not serve to limit the protection scope thereof.

| | Example 1 | Example 2 |
|---|---|---|
| Component | % by weight | % by weight |
| Water | 80.365 | 80.165 |
| Fenoxyethanol +paraben (Sharomix) | 1.000 | 1.000 |
| Citric acid | 0.005 | 0.005 |
| Disodium EDTA | 0.010 | 0.010 |
| Glycerin | 2.500 | 2.500 |
| Mineral oil | 8.000 | 8.000 |
| Keto-stearyl/keto-stearyl-glucoside-alcohol (Montanov 68) | 3.000 | 3.000 |
| Skeleton (Pripure 3759) | 0.010 | 0.010 |
| Cetyl palmitate | 3.0003.00 | |
| Clycerol 1-oleate (Monomuls 90 018) | 0.010 | 0.010 |
| Polyacrylamide/C13-C14 isoparafin / Laureth-7/ water (Sepigel 305) | 0.750 | 0.750 |
| Dihydroxyacetone | 1.000 | 1.200 |
| Fragrance (Linda Plus 3 SC3768D) | 0.350 | 0.350 |

The compositions corresponding to the preferred examples 1 and 2 shown above were analyzed, through corneometry for the hydration capability in different parts of the body, using untreated areas as control. The tests were carried out during a period of 24 hours, measurements having been taken after 30 min, 4 h, 7h and 24 h from application of the product. The comparison of the hydration that occurred after application of the product can be viewed in figure 6. As can bee observed, the application to the skin of the self-tanning composition of Example 1 showed, with respect to the control, an increase in hydration of 59.8%, 49.07%, 44.46% and 33.78% after 30 min, 4h, 7h and 24h of application of the product, respectively. The application of the self-tanning composition of Example 2 exhibited results similar to those of the self-tanning composition of Example 1 with an increase in hydration with respect to the control of 52.71 %, 42.96%, 30.76% and 25.66%, considering the same interval of measurement, respectively. These results show that the composition of the present invention exhibits moisturizing properties.

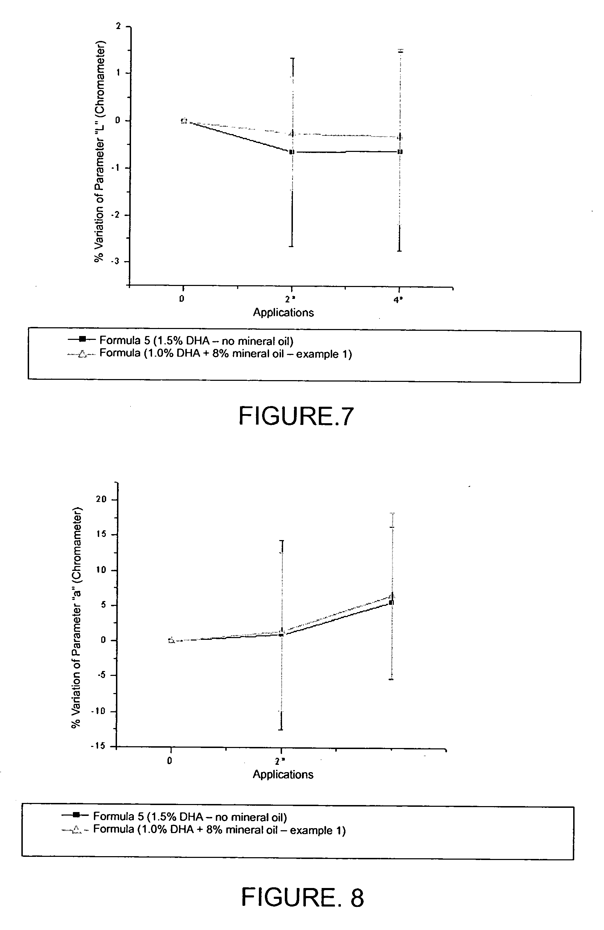

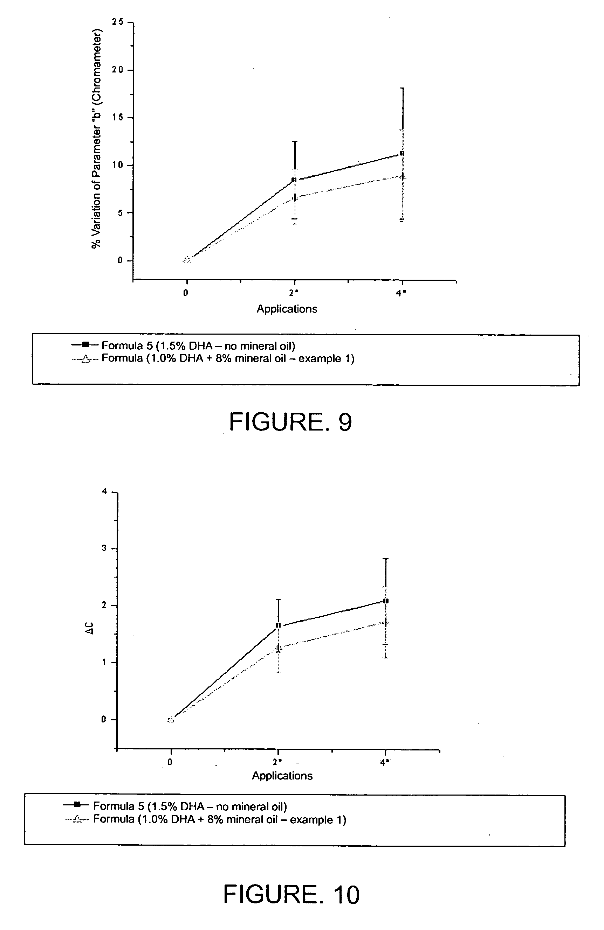

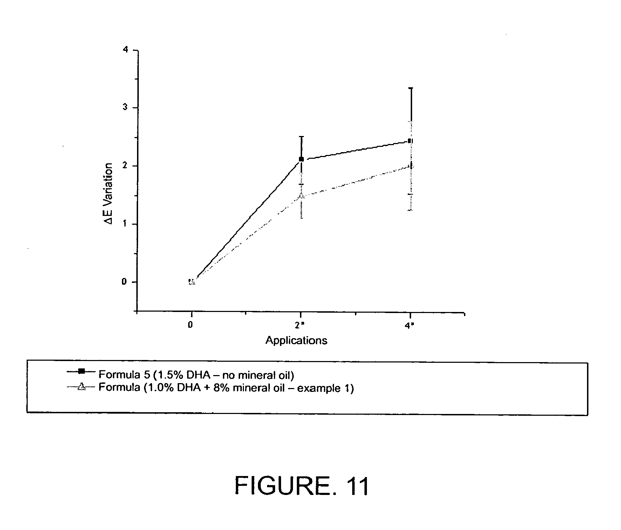

Aiming at a more specific comparison of the self-tanning composition of example 1 with respect to a self-tanning composition containing 1.5% DHA and not containing mineral oil (Formulation 5), one carried out measurements of the evolution of the color as a function of the days of application of the self-tanner, using colorimeter (Chromameter CR-200 - Minolta). One carried out, through colorimeter, measurements of variation of the yellow color (figure 7), variation of the red color (figure 8), variation of the yellow color (figure 9), chromatic variation (ΔC) considering the combined variation of the red and yellow colors (figure 10) and total combined variation (ΔE) considering the measurements of color brightness, variation of red color and variation of yellow color (figure 11).

The punctual values contained in the cited figures together with the corresponding standard diversion are shown in the tables below. Table 1 shows the values obtained upon analysis of variation of color brightness (figure 7). Table 2 shows the data obtained upon measurement of the variation of the red color (figure 8). Table 3 shows the values obtained upon measurement of variation of the yellow color (figure 9). Table 4 shows the data obtained upon measurement of the chromatic variation (ΔC) considering the combined variation of the red and yellow colors (figure 10). Table 5 shows the values of the data obtained upon total combined variation (ΔE) considering the measurements of the color brightness, variation of red color and variation of yellow color (figure 11).

**Table 1**

| | After 2^{nd} application | | After 4th application | |
|---|---|---|---|---|
| Color brightness | Middle | SD | Middle | SD |
| Formula 5 | -0.4613 | 2.0002 | -0.6274 | 2.1141 |
| Formula - example 1 | -0.247 | 1.2024 | -0.3052 | 1.8581 |

**Table 2**

| | After 2^{nd} application | | After 4^{th} application | |
|---|---|---|---|---|
| Color brightness | Middle | SD | Middle | SD |
| Formula 5 | 0.9911 | 13.423 | -5.5454 | 10.822 |
| Formula - example 1 | 1.4424 | 11.1846 | -6.5614 | 11.894 |

**Table 3**

| | After 2^{nd} application | | After 4^{th} application | |
|---|---|---|---|---|
| Color brightness | Middle | SD | Middle | SD |
| Formula 5 | 8.491 | 8.491 | 11.329 | 6.918 |
| Formula - example 1 | 6.7433 | 2.9111 | 8.97462 | 4.8563 |

**Table 4**

| | After 2^{nd} application | | After 4^{th} application | |
|---|---|---|---|---|
| AC | Middle | SD | Middle | SD |
| Formula 5 | 1.6542 | 0.4569 | 2.099 | 0.7535 |
| Formula - example 1 | 1.2835 | 0.4375 | 1.7238 | 0.6123 |

**Table 5**

| | After 2^{nd} application | | After 4^{th} application | |
|---|---|---|---|---|
| AE | Middle | SD | Middle | SD |
| Formula 5 | 2.1221 | 0.4057 | 2.4609 | 0.9152 |
| Formula - example 1 | 1.5028 | 0.3926 | 2.0312 | 0.7674 |

As can be observed in figures 7 - 11 and Tables 1 - 5 and, considering the standard diversion (SD) of the analyses carried out (shown in Tables 1 - 5), the self-tanning composition of Example 1 containing 1% by weight DHA and 8% by weight mineral oil exhibited results of color variation that were similar to those exhibited by composition 5. These results prove that the tanning effect of the joint presence of DHA and mineral oil can be used for obtaining self-tanning compositions with lower DHA concentration, without, however, diminishing the tanning effect achieved with such compositions.

Besides exhibiting results similar to those resulting from compositions with higher DHA concentrations, the composition described in the present invention does not have either the drawbacks of instability of compositions with high DHA concentrations or the drawbacks resulting from application of such products, like making the skin very dry and appearance of stains upon uneven application of the product or upon application thereof under keratinous regions of the body.

## Claims

1. A cosmetic and/or dermatological self-tanning composition, **characterized by** comprising, at most, 4% by weight DHA and, at least, 6% by weight mineral oil.

2. A cosmetic and/or dermatological self-tanning composition according to claim 1, **characterized by** comprising, at most, 2% by weight DHA and, at least, 8% by weight mineral oil.

3. A cosmetic and/or dermatological self-tanning composition according to claim 1 or 2, **characterized by** comprising 1.2% by weight DAH and 8% by weight mineral oil.

4. A cosmetic and/or dermatological self-tanning composition according to claim 3, **characterized by** being intended for application to normal-to-dark skin.

5. A cosmetic and/or dermatological self-tanning composition according to claim 1 or 2, **characterized by** comprising 1.0% by weight DHA and 8% by weight mineral oil.

6. A cosmetic and/or dermatological self-tanning composition according to claim 5, **characterized by** being intended for application to normal-to-pallor skin.

7. A cosmetic and/or dermatological self-tanning composition according to any of claims 1 - 6, **characterized by** comprising a wetting agent.

8. A cosmetic and/or dermatological self-tanning composition according to claim 7, **characterized in that** the wetting agent is glycerin.

9. A cosmetic and/or dermatological self-tanning composition according to claim 8, **characterized in that** the glycerin concentration is of 2.5% by weight.

10. A cosmetic and/or dermatological self-tanning composition according to any of claims 1 - 9, **characterized by** further comprising at least one component selected from carriers, preservatives, substances for pH adjustment and/or maintaince, viscosity agents, emulsifiers, emollients and perfumes.

11. Use of DHA in combination with mineral oil, **characterized by** being in the preparation of a cosmetic and/or dermatological composition as defined in any of claims 1 -10.

12. A skin self-tanning method, **characterized by** applying, onto the skin, the cosmetic and/or dermatological composition as defined in any of claims 1 -10.
